# EUROPEAN PATENT APPLICATION

(11) **EP 2 896 389 A1**
(43) Date of publication of application: **22.07.2015**
(21) Application number: 15388001.8
(22) Date of filing: 16.01.2015
(51) Int. Cl.: A61F 5/01

(54) **Support orthose system**

(30) Priority: 17.01.2014 DK 201400026
(71) Applicant: Bandagist Jan Nielsen A/S, 1360 Copenhagen K (DK)
(72) Inventor: Nielsen, Jan, 1360 Copenhagen K (DK)
(74) Representative: Larsen, Hans Ole

(57) **Abstract**

By designing an orthosis as a system comprising a relatively rigid sole (2) with flexible regions (11a-11e) extending across the sole (2), it will be possible to dimension it to control the movements of the foot (8) during walking.

It will be possible to ease the dorsal lift by providing the connecting member (7) between the sole (2) and the ankle cuff (1) with straps (4) of an elastic material, thereby obtaining an auxiliary force when the springy effect in the material is released in the dorsal lift.

This results in an improved walking motion as the sole supports the foot which is also assisted in the dorsal lift.

## Description

### The prior art

The invention relates to an orthotic system for supporting the walking ability of patients with reduced muscle function, said system comprising a sole which, by means of a brace around the ankle, is fixed to the foot.

Orthoses are used as support for patients with reduced or absent muscle function in knee and ankle following neurological damages, such as e.g. damages to the peripheral or to the central nervous system. Reduced or absent muscle function may also be due to acquired or inherited malalignments in the musculoskeletal system. The purpose of orthoses is thus to support the injured extremity as best possible in order to obtain an as normal as possible walking position and to relieve the patient's lack of proprioceptive sense.

Many types of support devices for knees, ankles and feet are known to relieve reduced or absent muscle function. The known support orthoses may be made from leather, metal or laminate. The orthoses may also be made from a combination of one or more of the above materials, and many orthoses are made from rigid or stiff materials.

Examples of orthotic systems are particularly known as ankle supports.

US 2011/0028877 A1 and US 2009/0247923 A1 disclose braces for supporting the ankle with the purpose of relieving the disadvantages of a weak ankle with respect to the walking motion and the dorsal lift.

However, there is a need for a foot support that can control/guide it in the desired direction of motion during walking.

As a foot support, DE 20 2011 107 040 U1 discloses an orthotic device comprising a spring ankle brace and a soft sole. However, this device lacks the ability to support a weak ankle musculature and thus to assist the foot in carrying out a springing push-off of the ground, just as the sole lacks the ability to control and support for obtaining the most advantageous walk.

Hence, the hitherto known orthoses only provide passive support to the patient and do not contribute to improving the patient's walking control or walking characteristics as well as dorsal lift.

In addition, the know orthoses are difficult to hide in the footwear, which is a strong wish among users of such support devices.

### The object of the invention

The object of the invention is therefore to provide a support orthosis offering the patient improved walking motion and dorsal lift.

This is obtained according to the invention if the sole, which covers the entire underside of the foot, is made from a relatively rigid material, if the front part of the sole is provided with flexible regions extending across the sole,

It will thus be possible to dimension the sole to support the walking ability as the rigid sole having one or more flexible regions, which will constitute bending lines, will allow for controlling the foot during the walking motion.

This walking motion usually happens by lifting first the heel, then the midfoot, and finally the toes, after which the toes are pressed towards the ground by the ankle musculature and thus act as an elastic spring assisting the motion of path. This movement usually follows the so-called motion of path line and will, by means of the sole according to the invention, be able to offer support in a hitherto unknown good manner. The bending of the sole will be placed and have a certain flexibility that will be able to control the motion of path and the dorsal lift of the foot.

By also providing bending lines in the heel portion of the sole, as specified in claim 2, the orthosis will be able to control the activation of the walking motion, as the sole by means of the bending provided in the heel will be able to control this initial movement in the desired direction.

The placement and flexibility of these regions will help control the foot, as specified in claim 3, as the sole will be fastened to the foot, which is thereby forced to follow the movements of the sole, as specified in claim 4.

By providing the connecting member between the sole and the ankle brace by means of elastic straps, as specified in claims 5 and 6, these will contribute to the push-off, and thus support the ankle musculature in the effort of lifting the foot, the so-called dorsal lift. This is due to the energy stored in the strap which is released in support of this lift.

### The drawing

An embodiment of the invention will be described in more detail below with reference to the drawing, in which
- Fig. 1: shows an ankle brace, connecting member and sole,
- Fig. 2: is an underside view of the sole,
- Fig. 3: is a sectional view of the sole seen in the direction III-III of Fig.2 and
- Figs. 4 and 5: show the orthosis fastened to the foot.

### Description of the embodiment

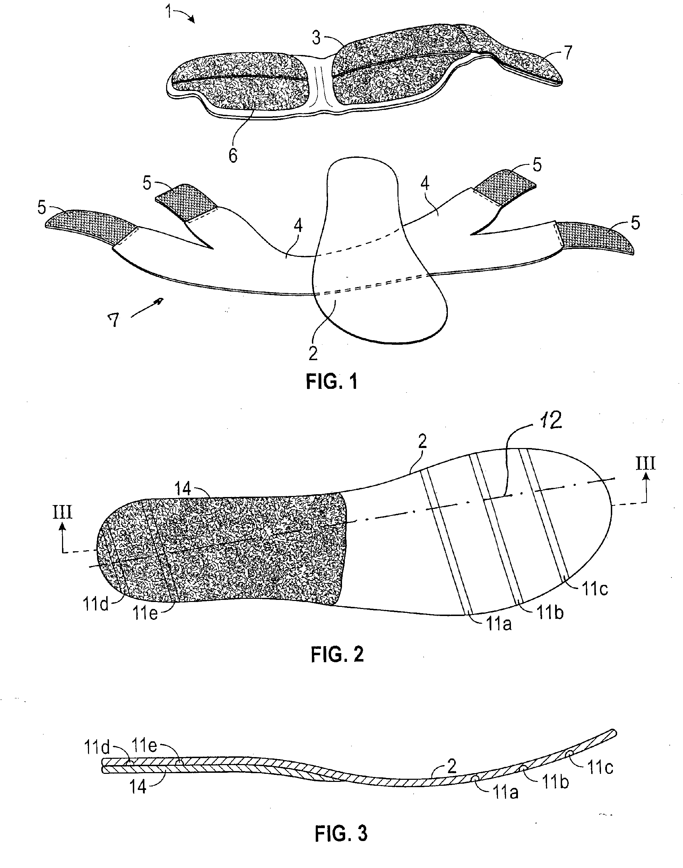

The orthotic system according to the invention comprises, as shown in Fig. 1, a sole 2, a connecting member 7 and an ankle cuff 1.

The sole 2 is, as shown in Figs. 2 and 3, shaped to cover the entire underside of the foot. The sole is furthermore, as indicated in Fig. 3, shaped according to the foot in such a manner that it fits tightly to the foot and supports it in its full extension.

Under the heel, a pad 14 may be adhered for achieving good walking comfort.

The sole 2 is preferably made from a composite material with reinforcing fibres such as carbon fibre or glass fibre. This provides a rigid sole that will act as good support for the foot.

As the foot must be supported in its movements during motion, which follows the dotted line 12 in the motion direction from the heel towards the toes, the sole needs to be able to control the foot, as it needs to be able to give way, i.e. to be flexible in certain places in certain directions.

In Figs. 2 and 3, these regions are marked by 11 a-11 e as they may be placed in both the front and in the heel portion of the sole.

These flexible regions are provided in the sole by interrupting the path of reinforcing fibres in the material in the places in question in such a manner that the basic material is non-reinforced in said regions.

This results in the sole 2 being able to give way and bend in these regions 11 a-11e and thus in the sole being adaptable according to the user's need for control of the motion path of the foot, i.e. the position and direction of the foot in relation to the motion path and support with respect to bending in the places in question.

In order to fasten the sole 2 to the foot and the ankle, the system includes an ankle cuff 1, which may be made from flexible synthetic material such as nylon. On the outer side, Velcro pads 3,6 are fastened just as a fastening 7 is provided, which may be a Velcro fastening or a clasp.

In order to connect the sole 2 and the cuff 1, a connecting member 7 is used, as shown in Fig. 1, said member comprising a portion 4 extending under the sole and being provided with straps which at the ends thereof are provided with Velcro pads 5 for fastening together with the pads 3,6 of the ankle cuff.

The connecting member 7 is principally made from an elastic synthetic material such as polyuretane, silicone, latex and the like for the best possible absorption and release of energy in the form of a spring release when the foot is near its dorsal lift, whereby the ankle musculature with support from the connecting member 7 will be assisted in the lift.

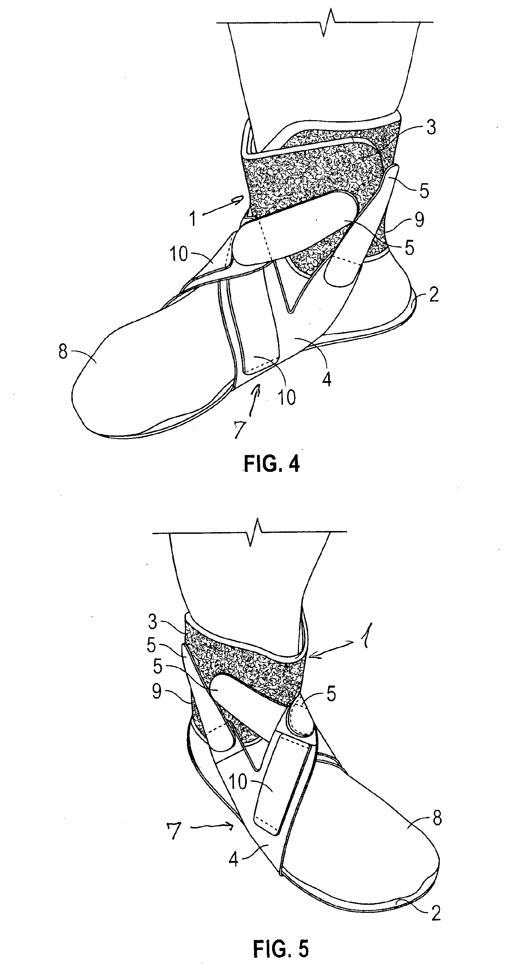

Figs. 4 and 5 show an orthosis according to the invention fastened to a foot 8.

First, the cuff 1 is arranged around the ankle, and the sole with the connecting member 7 is then fastened to the cuff as shown.

This orthosis can be made from materials so thin that it can be completely hidden in the shoe, which is considered an advantage to the user as it is thus rendered invisible.

The support orthosis provides a hitherto unknown good support during the walking motion as it can be dimensioned to support in several "levels" i.e. directions, just as the springy effect assists in easing the dorsal lift.

The system can be expanded with an electrical stimulation, e.g. of a pressure sensor embedded in the heel portion of the sole, which can signal a device arranged on the leg for electrical stimulation of the muscles. This allows for a good walking motion as the stimulation can be synchronised and thus allow even severely handicapped persons to obtain the best possible walking motion.

## Claims

1. An orthotic system for supporting the walking ability of patients with reduced muscle function, said system comprising a sole which by means of a brace around the ankle is fixed to the foot, **characterised in that** the sole (2), which covers the entire underside of the foot (8), is made from a relatively rigid material,
that the front part of the sole (2) is provided with flexible regions (11 a, 11 b, 11c) extending across the sole (2).

2. An orthotic system according to claim 1, **characterised in that** flexible regions (11d, 11e) are also provided across the rear end of the sole (2).

3. An orthotic system according to claims 1 and 2, **characterised in that** the flexible regions are directed in such a manner that they control the motion path of the foot (8) in such a manner that the sole (2) whilst bending during motion supports the movement of the foot (8) in desired directions.

4. An orthotic system according to claims 1-3, **characterised in that** the brace comprises a cuff (1) for fastening around the ankle as well as a connecting member (7) extending under the sole (2) and for fastening to the cuff (1).

5. An orthotic system according to claim 4, **characterised in that** the connecting member (7) comprises elastic straps (4) made from an energy-absorbing/energy-releasing material.

6. An orthotic system according to claim 5, **characterised in that** the connecting member (7) absorbs kinetic energy from the initial walking motion of the foot (8) and then releases the energy during the further motion path and dorsal lift.
